# EUROPEAN PATENT APPLICATION

(11) **EP 3 453 318 A1**
(43) Date of publication of application: **13.03.2019**
(21) Application number: 17190166.3
(22) Date of filing: 08.09.2017
(51) Int. Cl.: A61B 5/00, A61B 5/0488

(54) **AN APPARATUS AND METHOD FOR USE IN DETERMINING SENSITIVITY TO AN APPLIED STIMULUS**

(71) Applicant: Nokia Technologies Oy, 02610 Espoo (FI)
(72) Inventor: Müller, Kiti, 00430 Helsinki (FI); Ahmaniemi, Teemu, 00200 Helsinki (FI); Rajala, Satu, 02680 Espoo (FI); Kivioja, Jani, 2680 Espoo (FI)
(74) Representative: Potter Clarkson

(57) **Abstract**

An apparatus comprising:
at least one processor; and
at least one memory including computer program code, the at least one memory and computer program code configured to, with the at least one processor, cause the apparatus at least to:
control the magnitude of a stimulus applied by a stimulator to the skin of a human or animal body; and
receive an output from a sensor configured to detect the response of a muscle in the human or animal body to the applied stimulus, the sensor output in combination with the magnitude of the applied stimulus enabling the sensitivity of the human or animal body to the applied stimulus to be determined.

## Description

### Technical Field

The present disclosure relates to an apparatus and associated method which enable the sensitivity of a human or animal body to an applied stimulus to be determined using a stimulator and sensor. Certain embodiments specifically concern an apparatus configured to control the magnitude of a stimulus applied by a stimulator to the skin of a human or animal body, and receive an output from a sensor configured to detect the response of a muscle in the human or animal body to the applied stimulus. In these embodiments, the sensor output in combination with the magnitude of the applied stimulus enable the sensitivity of the human or animal body to the applied stimulus to be determined.

### Background

Research is currently being done to develop new medical devices and associated techniques.

The listing or discussion of a prior-published document or any background in this specification should not necessarily be taken as an acknowledgement that the document or background is part of the state of the art or is common general knowledge.

### Summary

According to a first aspect, there is provided an apparatus comprising:
at least one processor; and
at least one memory including computer program code, the at least one memory and computer program code configured to, with the at least one processor, cause the apparatus at least to:
   control the magnitude of a stimulus applied by a stimulator to the skin of a human or animal body; and
   receive an output from a sensor configured to detect the response of a muscle in the human or animal body to the applied stimulus, the sensor output in combination with the magnitude of the applied stimulus enabling the sensitivity of the human or animal body to the applied stimulus to be determined.

The apparatus may be configured to control the magnitude of the applied stimulus by one or more of increasing and decreasing the magnitude over time to enable determination of a respective sensing and de-sensing threshold of the human or animal body to the applied stimulus.

The apparatus may be configured to increase or decrease the magnitude linearly, exponentially or step-wise.

The apparatus may be configured to determine one or more of the sensing and de-sensing threshold of the human or animal body based on the sensor output and the magnitude of the applied stimulus.

The apparatus may be configured to:
compare one or more of the sensing and de-sensing threshold with a reference threshold; and
identify any difference between the sensing/de-sensing threshold and the reference threshold as an indication of the sensitivity of the human or animal body to the applied stimulus.

The apparatus may be configured to:
determine one or more of a stimulator signal and a sensor baseline signal; and
remove the stimulator signal and/or sensor baseline signal from the sensor output.

The applied stimulus may comprise touch or vibration, and the stimulator may comprise at least one actuator configured to apply pressure or vibrations to the skin of the human or animal body.

The applied stimulus may comprise heat, and the stimulator may comprise a heating element configured to apply heat to the skin of the human or animal body.

The muscle response may comprise activation of the muscle, and the sensor may comprise first and second surface electromyography electrodes and a voltmeter configured to measure the potential difference therebetween to detect said activation.

The muscle response may comprise movement of at least one of the muscle and the human or animal body, and the sensor may comprise one or more force sensors to detect said movement.

At least one of the stimulator and sensor may be provided on one or more platforms configured to support the human or animal body.

The apparatus may comprise one or more of the stimulator and sensor.

The apparatus may comprise a plurality of stimulator-sensor pairs. Two or more of the stimulator-sensor pairs may be the same or different.

The stimulus applied by the stimulator to the skin of the human or animal body may be a non-invasive stimulus. The term "non-invasive" in this respect may be taken to mean that the stimulus is applied to the exterior of the human or animal body rather than the interior.

The stimulator may be configured to apply the stimulus to one or more of the skin surface, epidermis, dermis and hypodermis.

The apparatus may be configured to generate one or more signals indicative of the sensitivity of the human or animal body to the applied stimulus.

The one or more generated signals may comprise one or more of the magnitude of the applied stimulus, the sensor output, the sensing threshold, the de-sensing threshold, and any difference between the sensing/de-sensing threshold and the corresponding threshold designated as being representative of a healthy human or animal body.

The apparatus may be configured to control the applied stimulus such that the stimulus is applied continuously, periodically or aperiodically to the skin of the human or animal body during detection of the muscle response by the sensor.

The human or animal body may be one or more of the following human or animal body parts: foot, hand, arm, leg and paw.

The apparatus may be at least one of an electronic device, a portable electronic device, a medical device and a module for one or more of the same.

According to a further aspect, there is provided a method comprising:
controlling the magnitude of a stimulus applied by a stimulator to the skin of a human or animal body; and
receiving an output from a sensor configured to detect the response of a muscle in the human or animal body to the applied stimulus, the sensor output in combination with the magnitude of the applied stimulus enabling the sensitivity of the human or animal body to the applied stimulus to be determined.

The method may comprise controlling the magnitude of the applied stimulus by one or more of increasing and decreasing the magnitude over time to enable determination of a respective sensing and de-sensing threshold of the human or animal body to the applied stimulus.

The method may comprise increasing or decreasing the magnitude linearly, exponentially or step-wise.

The method may comprise determining one or more of the sensing and de-sensing threshold of the human or animal body based on the sensor output and the magnitude of the applied stimulus.

The method may comprise:
comparing one or more of the sensing and de-sensing threshold with a reference threshold; and
identifying any difference between the sensing/de-sensing threshold and the reference threshold as an indication of the sensitivity of the human or animal body to the applied stimulus.

The method may comprise:
determining one or more of a stimulator signal and a sensor baseline signal; and
removing the stimulator signal and/or sensor baseline signal from the sensor output.

The method may comprise generating one or more signals indicative of the sensitivity of the human or animal body to the applied stimulus.

The method may comprise controlling the applied stimulus such that the stimulus is applied continuously, periodically or aperiodically to the skin of the human or animal body during detection of the muscle response by the sensor.

According to a further aspect, there is provided an apparatus comprising:
at least one processor; and
at least one memory including computer program code, the at least one memory and computer program code configured to, with the at least one processor, cause the apparatus at least to:
   determine the magnitude of a stimulus applied by a stimulator to the skin of a human or animal body; and
   receive an output from a sensor configured to detect the response of a muscle in the human or animal body to the applied stimulus, the sensor output in combination with the magnitude of the applied stimulus enabling the sensitivity of the human or animal body to the applied stimulus to be determined.

According to a further aspect, there is provided a method comprising:
determining the magnitude of a stimulus applied by a stimulator to the skin of a human or animal body; and
receiving an output from a sensor configured to detect the response of a muscle in the human or animal body to the applied stimulus, the sensor output in combination with the magnitude of the applied stimulus enabling the sensitivity of the human or animal body to the applied stimulus to be determined.

The steps of any method disclosed herein do not have to be performed in the exact order disclosed, unless explicitly stated or understood by the skilled person.

Corresponding computer programs for implementing one or more steps of the methods disclosed herein are also within the present disclosure and are encompassed by one or more of the described example embodiments.

One or more of the computer programs may, when run on a computer, cause the computer to configure any apparatus, including a battery, circuit, controller, or device disclosed herein or perform any method disclosed herein. One or more of the computer programs may be software implementations, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

One or more of the computer programs may be provided on a computer readable medium, which may be a physical computer readable medium such as a disc or a memory device, or may be embodied as a transient signal. Such a transient signal may be a network download, including an internet download.

The present disclosure includes one or more corresponding aspects, example embodiments or features in isolation or in various combinations whether or not specifically stated (including claimed) in that combination or in isolation. Corresponding means for performing one or more of the discussed functions are also within the present disclosure.

The above summary is intended to be merely exemplary and non-limiting.

### Brief Description of the Figures

A description is now given, by way of example only, with reference to the accompanying drawings, in which:-
Figure 1 shows an apparatus configured to control the stimulus applied by a first stimulator;
Figure 2a shows how the applied stimulus varies over time;
Figure 2b shows how the resulting muscle response varies over time;
Figure 2c shows the muscle response of Figure 2b after removal of unwanted signal components;
Figure 3 shows an apparatus configured to control the stimulus applied by a second stimulator;
Figure 4 shows a platform comprising the stimulator and sensor of Figure 1;
Figure 5 shows another apparatus;
Figure 6 shows a method of using an apparatus; and
Figure 7 shows a computer-readable medium comprising a computer program configured to perform, control or enable the method of Figure 6.

### Description of Specific Aspects/Embodiments

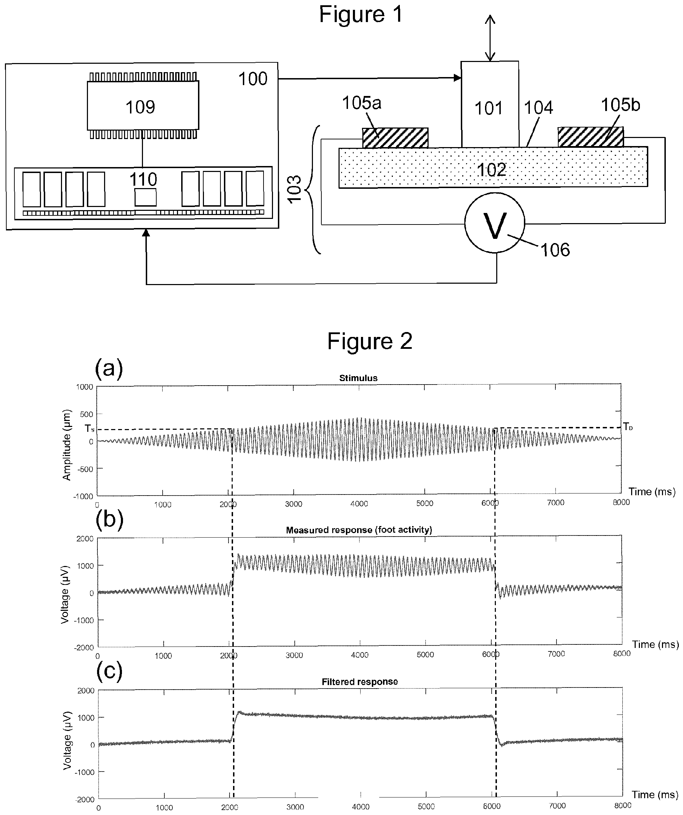

Figure 1 shows one example of an apparatus 100. The apparatus 100 comprises at least one processor 109 and at least one memory 110 including computer program code. The at least one memory 110 and computer program code are configured to, with the at least one processor 109, cause the apparatus 100 at least to control the magnitude of a stimulus applied by a stimulator 101 to the skin 104 of a human or animal body 102, and receive an output from a sensor 103 configured to detect the response of a muscle in the human or animal body 102 to the applied stimulus. The sensor output in combination with the magnitude of the applied stimulus enables the sensitivity of the human or animal body 102 to the applied stimulus to be determined. One or both of the stimulator 101 and sensor 103 may or may not form part of the apparatus 100.

The term "human or animal body" may be taken to mean one or more human or animal body parts (e.g. foot, hand, arm, leg and/or paw). In addition, the stimulator 101 may be configured to apply the stimulus locally (i.e. to a specific area) or globally (i.e. to an entire/general area) to the skin 104 of the human or animal body 102. For example, the stimulus may be applied globally to the entire sole of a patient's foot or locally to one or more of the medial plantar, lateral plantar, tibial, sapheneous and sural nerve areas of the sole. Furthermore, the stimulator 101 may be configured to apply the stimulus to one or more of the skin surface, epidermis, dermis and hypodermis.

In this example, the applied stimulus comprises vibration, and the stimulator 101 comprises at least one actuator (e.g. an electromechanical or piezoelectric actuator) configured to apply vibrations to the skin 104 of the human or animal body 102. In another example, however, the applied stimulus may comprise touch, and the actuator may be configured to apply pressure to the skin 104 of the human or animal body 102.

In addition, the sensor 103 comprises first 105a and second 105b surface electromyography electrodes (although there could be more than two electrodes), and a voltmeter 106 configured to measure the potential difference therebetween, to detect activation of the muscle in response to the applied stimulus. The surface electromyography electrodes 105a,b may be attached to the human or animal body 102 (e.g. using a strap or adhesive) or provided on a platform configured to support the human or animal body 102 (described in more detail later). Additionally or alternatively, the sensor 103 may comprise one or more force sensors. In this scenario, the force sensors may be used to detect any movement of the human or animal body 102 in response to the applied stimulus.

Figure 2a is a graph showing how the applied stimulus may vary over time. In this example, the stimulator 101 is controlled to continuously and linearly increase the magnitude of the applied stimulus over time to enable determination of the minimum magnitude at which the muscle is activated by the applied stimulus. Here, the magnitude of the applied stimulus is the amplitude of vibration applied to the skin 104 by the actuator 101, and the periodic nature of the graph is representative of the applied vibration. At the minimum detectable magnitude of applied stimulus, the stimulus is sensed by nerve receptors in the skin 104 which in turn cause muscle activation.

Figure 2b is an example graph showing the muscle response in the form of voltage measurements made using the first 105a and second 105b surface electromyography electrodes. The muscle response depicted in the graph comprises a higher frequency component and a lower frequency component. The higher frequency component represents a stimulator signal caused by the applied vibrations which is picked up by the electrodes 105a,b, and the lower frequency component represents the muscle response to the applied vibrations. As can be seen, activation of the muscle first occurs after -2.1 seconds when the amplitude of vibration is increased to a sensing threshold T_{S} of the human or animal body 102. The term "sensing threshold" may be taken to mean the threshold where muscle activation begins when the magnitude of the stimulus is increased.

In this example, the stimulator 101 is also controlled to continuously and linearly decrease the magnitude of the applied stimulus to enable determination of the magnitude at which the muscle activation stops. It can be seen in Figure 2b that activation of the muscle stops after -6.1 seconds when the amplitude of vibration is decreased to a de-sensing threshold T_{D} of the human or animal body 102. The term "de-sensing threshold" may be taken to mean the threshold where muscle activation stops when the magnitude of the stimulus is decreased.

It is worth noting here that the de-sensing threshold T_{D} can be higher, lower or substantially the same as the sensing threshold Ts. For example, the skin's nerve receptors can become pre-sensitised or de-sensitised to the applied stimulus. Pre-sensitisation refers to priming of the receptors by the applied stimulus as the magnitude is increased to the sensing threshold T_{S} which can result in an increase in sensitivity and a corresponding decrease in the sensing threshold Ts. In contrast, de-sensitisation refers to a decrease in sensitivity and a corresponding increase in the de-sensing threshold T_{D} caused by prolonged stimulation of the receptors.

In some cases (although not shown in Figure 2), the stimulator 101 may be controlled to further increase the magnitude of the applied stimulus to enable determination of the minimum magnitude at which the muscle (and/or the human or animal body 102) exhibits movement in response to the applied stimulus. Movement of a muscle or body 102 occurs when the magnitude of the stimulus is sufficiently large that the muscle responds with a twitch or jerk reaction (also called a spontaneous yield reaction or reflex) to the stimulus rather than just activation of a motor unit within the muscle (which typically occurs at a lower magnitude of applied stimulus). Furthermore, whilst muscle activation may feel rather faint (or even mildly pleasurable) to the patient, movement of the muscle or body 102 may be accompanied by pain or discomfort (somewhat similar to an electric shock).

Although the magnitude of the applied stimulus is increased and decreased linearly in Figure 2a, it could alternatively be varied exponentially or step-wise. In addition, rather than applying the stimulus continuously during detection of the response by the sensor 103, it could be applied periodically (i.e. in bursts) or aperiodically. A periodic stimulus may reduce receptor adaptation of the muscle to the applied stimulus resulting in a smaller difference between the sensing T_{S} and de-sensing T_{D} thresholds when compared with a continuous stimulus. Also, the cycle shown in Figures 2a and 2b may be repeated any number of times in order to obtain average or median values of the sensing T_{S} and/or de-sensing T_{D} threshold and exclude any outliers or erroneous measurements.

The apparatus 100 may be configured to generate one or more signals indicative of the sensitivity of the human or animal body 102 to the applied stimulus. For instance, the one or more generated signals may comprise the magnitude of the applied stimulus and the sensor output from which the sensing T_{S} and/or de-sensing T_{D} threshold can be determined (e.g. by a user of the apparatus 100). Alternatively, the apparatus 100 may be configured to determine one or more of the sensing T_{S} and de-sensing T_{D} threshold of the human or animal body 102 based on the sensor output and the magnitude of the applied stimulus, and the one or more generated signals may comprise the determined sensing T_{S} and/or de-sending T_{D} threshold (with or without the sensor output and magnitude of applied stimulus).

In some examples, the apparatus 100 may be configured to compare one or more of the sensing T_{S} and de-sensing T_{D} threshold with a reference threshold, and identify any difference between the sensing/de-sending threshold and the reference threshold as an indication of the sensitivity of the human or animal body 102 to the applied stimulus. For instance, the reference threshold may be a corresponding sensing or de-sensing threshold designated as being representative of a healthy or unhealthy human or animal body. In these examples, the one or more generated signals may comprise the identified difference between the sensing/de-sensing threshold and the corresponding threshold (with or without the sensor output, magnitude of applied stimulus and sensing/de-sending threshold).

The apparatus 100 may be configured to present the one or more generated signals (which may be audible and/or visual signals), or signals derived from the one or more generated signals, to a user of the apparatus 100 (e.g. a patient or medical practitioner) so that he/she can determine the sensitivity of the human or animal body 102 to the applied stimulus manually. The derived signals may be different formats of the one or more generated signals, such as textual or graphical representations of numeric data. Additionally or alternatively, the apparatus 100 may be configured to transmit the one or more generated signals (or the derived signals) to a remote apparatus so that the sensitivity of the human or animal body 102 to the applied stimulus can be determined remotely by the remote apparatus or a remote person. The latter scenario may be beneficial for remote health monitoring of patients by medical practitioners.

In some cases, the apparatus 100 may be configured to determine one or more of a stimulator signal and a sensor baseline signal (which are unwanted signal artefacts), and remove the stimulator signal and/or sensor baseline signal from the sensor output. The sensor baseline signal represents the background muscle activation or movement detected before the stimulus has been applied. The stimulator signal and sensor baseline signal typically have frequencies which are substantially uniform and distinct from the muscle response, which facilitates their removal.

Figure 2c shows the detected response after the apparatus 100 has removed the stimulator signal and sensor baseline signal. As can be seen, removal of such unwanted signal components results in a cleaner trace which more accurately reflects the muscle response to the applied stimulus. Here the term "remove" may be taken to mean that the unwanted signal components are at least partially decreased in the detected response.

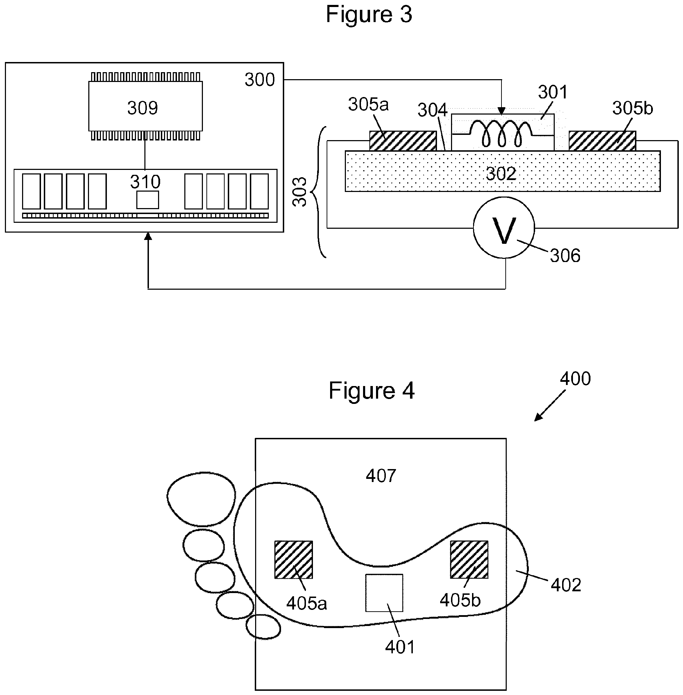

Figure 3 shows an apparatus 300 configured to control the stimulus applied by a different type of stimulator 301. In this example, the applied stimulus comprises heat, and the stimulator 301 comprises a heating element (e.g. a resistive heating element) configured to apply heat to the human or animal body 302. As per the example shown in Figure 1, the apparatus 300 comprises at least one processor 309 and at least one memory 310 including computer program code, and the sensor 303 comprises first 305a and second 305b surface electromyography electrodes and a voltmeter 306 configured to measure the potential difference therebetween to detect activation of the muscle in response to the applied stimulus. One or both of the stimulator 301 and sensor 303 may or may not form part of the apparatus 300.

As per the graph of Figure 2a, the magnitude of the applied stimulus may be increased or decreased to determine the sensing/de-sensing thresholds. In this case, the magnitude of the stimulus is the amount of heat applied to the skin 304 by the heating element 301 (which may be determined by the temperature of the skin 304 or heating element 301).

Figure 4 shows another apparatus 400. In this example, the stimulator and sensor are provided on one or more platforms 407 configured to support the human or animal body 402 (e.g. each platform 407 configured to support a different body part). As shown in Figure 4, a platform 407 may comprise an actuator 401 and surface electromyography electrodes 405a,b such that the response of a muscle on the sole of a patient's foot 402 can be detected when the patient stands on the platform 407.

In some cases, the apparatus described herein may be configured to enable the response of multiple muscles to be detected at the same time. This may be achieved if the apparatus comprises a plurality of independent stimulator-sensor pairs. For example, the stimulator-sensor pairs may be suitably arranged in different regions of the upper surface of the platform in Figure 4 corresponding to the relevant muscles in the human or animal body (although the platform is not essential). In addition, different types of stimulator and/or sensor may be used to test the response of the same or different muscles to more than one stimuli. Determining the sensitivity to different stimuli can be useful because some patients lose sensitivity to one stimulus but retain sensitivity to other stimuli. Alternatively, the same type of stimulator and/or sensor may be used to test the response of different muscles to the same stimulus.

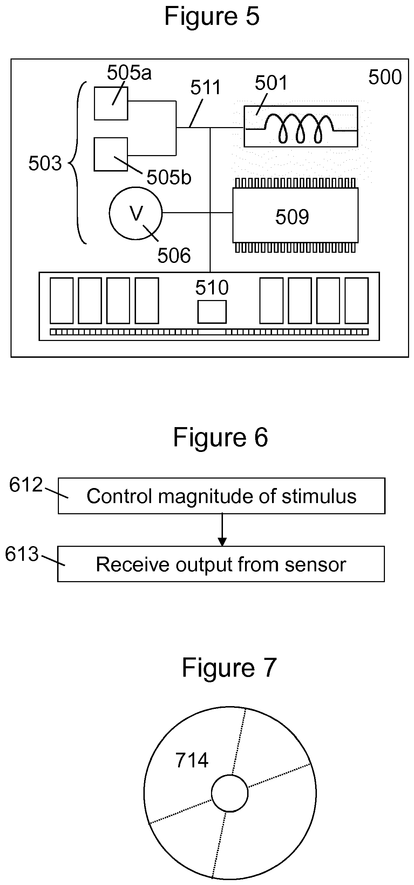

Figure 5 shows another apparatus 500. The apparatus 500 may be at least one of an electronic device, a portable electronic device, a medical device and a module for one or more of the same. In the example shown, the apparatus 500 comprises a stimulator 501 (in this case, a heating element), a sensor 503 (in this case, surface electromyography electrodes 505a,b and a voltmeter 506), a processor 509 and a storage medium 510, which are electrically connected to one another by a data bus 511.

The stimulator 501 is configured to apply a stimulus to the skin of the human or animal body; the sensor 503 is configured to detect the response of a muscle in the human or animal body to the applied stimulus; and the processor 509 (with the storage medium 510 and appropriate computer program code) is configured to control the magnitude of the stimulus applied by the stimulator 501 and receive an output from the sensor 503.

The processor 509 may be configured for general operation of the apparatus 500 by providing signalling to, and receiving signalling from, the other components to manage their operation. The storage medium 510 (or a separate storage medium) is configured to store computer code configured to perform, control or enable operation of the apparatus 500. The storage medium 510 may also be configured to store settings for the other components. The processor 509 may access the storage medium 510 to retrieve the component settings in order to manage the operation of the other components. For example, the storage medium 510 may store settings for the stimulator 501, and the processor 509 may utilise these settings to generate a control signal to control the magnitude of the applied stimulus.

The processor 509 may be a microprocessor, including an Application Specific Integrated Circuit (ASIC). The storage medium 510 may be a temporary storage medium such as a volatile random access memory. On the other hand, the storage medium 510 may be a permanent storage medium such as a hard disk drive, a flash memory, or a non-volatile random access memory.

Although not shown, the apparatus 500 may also comprise a power supply (e.g. comprising one or more of a mains supply, a primary battery, a secondary battery, a capacitor, a supercapacitor and a battery-capacitor hybrid) configured to provide each of the components with electrical power to enable their functionality.

The apparatus 500 may also comprise an electronic display (e.g. an LED, LCD or plasma display) configured to visually present the one or more generated/derived signals to a user of the apparatus 500, a loudspeaker configured to aurally present the one or more generated/derived signals to a user of the apparatus 500 and/or a transmitter configured to transmit the one or more generated/derived signals to a remote apparatus 500. In this scenario, the one or more generated signals may comprise one or more of the magnitude of the applied stimulus, the sensor output, the sensing threshold, the de-sensing threshold, and any difference between the sensing/de-sensing threshold and the corresponding threshold designated as being representative of a healthy human or animal body (as described previously).

In some examples, the apparatus described herein may be configured to determine the magnitude of the applied stimulus rather than control it. For instance, the apparatus may be connectable (e.g. via a wired or wireless connection) to an existing stimulator to obtain the magnitude of the applied stimulus for subsequent analysis in combination with the sensor output.

Figure 6 shows schematically the main steps 612-613 of a method of using the present apparatus. The method generally comprises: controlling the magnitude of a stimulus applied by a stimulator to the skin of a human or animal body 612; and receiving an output from a sensor configured to detect the response of a muscle in the human or animal body to the applied stimulus 613.

Figure 7 illustrates schematically a computer/processor readable medium 714 providing a computer program according to one embodiment. The computer program may comprise computer code configured to perform, control or enable one or more of the method steps 612-613 of Figure 6 using an apparatus 100, 300, 400, 500 described herein. In this example, the computer/processor readable medium 714 is a disc such as a digital versatile disc (DVD) or a compact disc (CD). In other embodiments, the computer/processor readable medium 714 may be any medium that has been programmed in such a way as to carry out an inventive function. The computer/processor readable medium 714 may be a removable memory device such as a memory stick or memory card (SD, mini SD, micro SD or nano SD).

A technical effect of one or more examples described herein is that the requirement for a patient to provide oral feedback to a medical practitioner during a medical examination is reduced, and therefore a more objective and/or quantitative response may be provided. This aspect also renders the one or more examples suitable for use with animals in veterinary medicine. Furthermore, another technical effect is that greater control over the application of the stimulus may be achieved. This enables more consistency between medical examinations and greater reproducibility in the results. In combination, therefore, a more accurate and reliable medical examination may be obtained than with existing techniques.

Other embodiments depicted in the figures have been provided with reference numerals that correspond to similar features of earlier described embodiments. For example, feature number 1 can also correspond to numbers 101, 201, 301 etc. These numbered features may appear in the figures but may not have been directly referred to within the description of these particular embodiments. These have still been provided in the figures to aid understanding of the further embodiments, particularly in relation to the features of similar earlier described embodiments.

It will be appreciated to the skilled reader that any mentioned apparatus/device and/or other features of particular mentioned apparatus/device may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on one or more memories/processors/functional units.

In some embodiments, a particular mentioned apparatus/device may be pre-programmed with the appropriate software to carry out desired operations, and wherein the appropriate software can be enabled for use by a user downloading a "key", for example, to unlock/enable the software and its associated functionality. Advantages associated with such embodiments can include a reduced requirement to download data when further functionality is required for a device, and this can be useful in examples where a device is perceived to have sufficient capacity to store such pre-programmed software for functionality that may not be enabled by a user.

It will be appreciated that any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

It will be appreciated that any "computer" described herein can comprise a collection of one or more individual processors/processing elements that may or may not be located on the same circuit board, or the same region/position of a circuit board or even the same device. In some embodiments one or more of any mentioned processors may be distributed over a plurality of devices. The same or different processor/processing elements may perform one or more functions described herein.

It will be appreciated that the term "signalling" may refer to one or more signals transmitted as a series of transmitted and/or received signals. The series of signals may comprise one, two, three, four or even more individual signal components or distinct signals to make up said signalling. Some or all of these individual signals may be transmitted/received simultaneously, in sequence, and/or such that they temporally overlap one another.

With reference to any discussion of any mentioned computer and/or processor and memory (e.g. including ROM, CD-ROM etc), these may comprise a computer processor, Application Specific Integrated Circuit (ASIC), field-programmable gate array (FPGA), and/or other hardware components that have been programmed in such a way to carry out the inventive function.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed aspects/embodiments may consist of any such individual feature or combination of features. In view of the foregoing description it will be evident to a person skilled in the art that various modifications may be made within the scope of the disclosure.

While there have been shown and described and pointed out fundamental novel features as applied to different embodiments thereof, it will be understood that various omissions and substitutions and changes in the form and details of the devices and methods described may be made by those skilled in the art without departing from the spirit of the invention. For example, it is expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or embodiment may be incorporated in any other disclosed or described or suggested form or embodiment as a general matter of design choice. Furthermore, in the claims means-plus-function clauses are intended to cover the structures described herein as performing the recited function and not only structural equivalents, but also equivalent structures. Thus although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. An apparatus comprising:
at least one processor; and
at least one memory including computer program code, the at least one memory and computer program code configured to, with the at least one processor, cause the apparatus at least to:
control the magnitude of a stimulus applied by a stimulator to the skin of a human or animal body; and
receive an output from a sensor configured to detect the response of a muscle in the human or animal body to the applied stimulus, the sensor output in combination with the magnitude of the applied stimulus enabling the sensitivity of the human or animal body to the applied stimulus to be determined.

2. The apparatus of claim 1, wherein the apparatus is configured to control the magnitude of the applied stimulus by one or more of increasing and decreasing the magnitude over time to enable determination of a respective sensing and de-sensing threshold of the human or animal body to the applied stimulus.

3. The apparatus of claim 2, wherein the apparatus is configured to increase or decrease the magnitude linearly, exponentially or step-wise.

4. The apparatus of claim 2 or 3, wherein the apparatus is configured to determine one or more of the sensing and de-sensing threshold of the human or animal body based on the sensor output and the magnitude of the applied stimulus.

5. The apparatus of any of claims 2 to 4, wherein the apparatus is configured to:
compare one or more of the sensing and de-sensing threshold with a reference threshold; and
identify any difference between the sensing/de-sensing threshold and the reference threshold as an indication of the sensitivity of the human or animal body to the applied stimulus.

6. The apparatus of any preceding claim, wherein the apparatus is configured to:
determine one or more of a stimulator signal and a sensor baseline signal; and
remove the stimulator signal and/or sensor baseline signal from the sensor output.

7. The apparatus of any preceding claim, wherein the apparatus is configured to control the applied stimulus such that the stimulus is applied continuously, periodically or aperiodically to the skin of the human or animal body during detection of the muscle response by the sensor.

8. The apparatus of any preceding claim, wherein the applied stimulus comprises touch or vibration, and the stimulator comprises at least one actuator configured to apply pressure or vibrations to the skin of the human or animal body.

9. The apparatus of any preceding claim, wherein the applied stimulus comprises heat, and the stimulator comprises a heating element configured to apply heat to the skin of the human or animal body.

10. The apparatus of any preceding claim, wherein the muscle response comprises activation of the muscle, and wherein the sensor comprises first and second surface electromyography electrodes and a voltmeter configured to measure the potential difference therebetween to detect said activation.

11. The apparatus of any preceding claim, wherein the muscle response comprises movement of at least one of the muscle and the human or animal body, and wherein the sensor comprises one or more force sensors configured to detect said movement.

12. The apparatus of any preceding claim, wherein at least one of the stimulator and sensor are provided on one or more platforms configured to support the human or animal body.

13. The apparatus of any preceding claim, wherein the apparatus comprises one or more of the stimulator and sensor.

14. A method comprising:
controlling the magnitude of a stimulus applied by a stimulator to the skin of a human or animal body; and
receiving an output from a sensor configured to detect the response of a muscle in the human or animal body to the applied stimulus, the sensor output in combination with the magnitude of the applied stimulus enabling the sensitivity of the human or animal body to the applied stimulus to be determined.

15. A computer program comprising computer code configured to control the method of claim 14.
